# EUROPEAN PATENT APPLICATION

(11) **EP 1 932 542 A1**
(43) Date of publication of application: **18.06.2008**
(21) Application number: 06026072.6
(22) Date of filing: 15.12.2006
(51) Int. Cl.: A61K 45/06, A61P 3/10, A23L 1/303, A23L 1/305

(54) **Antioxidant composition and its use in diabetes**

(71) Applicant: TIMA Foundation, 9496 Balzers (LI)
(72) Inventor: Graf Matuschka von Greiffenclau, Markus, 9220 Bischofszell (CH); Inufusa, Haruhiko, Chuouku Osaka-city 542-0081 (JP)
(74) Representative: Dehmel, Albrecht

(57) **Abstract**

The present invention relates to a composition comprising Vitamin C, glutamic acid and/or glutamine, cysteine, riboflavin, succinic acid, fumaric acid, coenzyme Q10 and niacin. The composition can be used as food supplement and/or medicament for prophylaxis or treatment of several disease conditions. Consequently, the present invention is also directed to a pharmaceutical composition for the use in human and veterinary medicine.

## Description

The present invention relates to a composition comprising Vitamin C, glutamic acid and/or glutamine, cysteine, riboflavin, succinic acid, fumaric acid, coenzyme Q10 and niacin. The composition can be used as food supplement and/or medicament for prophylaxis or treatment of several disease conditions. Consequently, the present invention is also directed to a pharmaceutical composition for the use in human and veterinary medicine.

Hyperglycaemia is a condition associated with various diseases conditions. One of the most prominent diseases states with hyperglycaemia as symptom is Diabetes mellitus. Diabetes mellitus is a systemic disease which starts with continuous high levels of blood glucose. A healthy subject controls the levels of blood glucose by the hormone insulin and keeps the blood glucose levels in between about 80 to about 100 mg/dl in hungry status. In patients with diabetes the levels can exceed 125 mg/dl.

Since the first therapeutic use of insulin diabetes mellitus has been a treatable but chronic disease condition and the main health risks are the characteristic long-term complications associated with the disease. These include an increased risk for cardiovascular diseases, chronic renal failure, retinal damage possibly resulting in blindness, nerve damage, erectile dysfunction (impotence) and gangrene with risk of amputation. A test usually used to test for diabetes, but also for insulin resistance is the glucose tolerance test.

Glucose tolerance test in medical practice is the administration of glucose to determine how quickly it is cleared from the blood. The glucose is most often given orally so the common test is technically an oral glucose tolerance test (OGTT).

Meanwhile, there are several drugs for the treatment of diabetes. Oral drugs for the treatment of diabetes mellitus are classified in four categories. Within the first category are inhibitors of glucose absorption e.g. alpha glucosidase inhibitors. In the second category are inhibitors of glucose production in the liver e.g. biguanide. The third category are drugs improving the insulin sensitivity of the body e.g. thiazolidine derivatives. To the fourth category belong stimulators of insulin secretion e.g. sulfonyl urea or tenaglinid. These drugs are essentially dependent on the effects of insulin controlling glucose metabolism.

For improvement of the health status of an individual in general numerous food supplements are available. Usually they are based on the use of Vitamins and other metabolically relevant substances. Some of these also claim to be beneficial for patients suffering from diabetes or diabetes related conditions.

For example, in US Patent application US 2002155163 it has been proposed that a daily multi-Vitamin and mineral combination can be of use in the adjunct care of humans with diabetes. The composition comprises thiamin, riboflavin, niacin, Vitamin B6, folate, Vitamin B12, biotin, pantothenic acid, choline, inositol, para-amino benzoic acid, Vitamin C, calcium, magnesium, iodine, selenium, manganese, chromium, molybdenum, boron, zinc, potassium, silicon, sulfur, vanadium, citrus bioflavonoid complex, hesperidin complex, rutin, Vitamin A, Vitamin D, Vitamin E, lycopene, lutein, coenzyme Q10 and alpha-lipoic acid.

In another example it has been proposed to use for treatment of diabetic neuropathy a pharmaceutical composition comprising as active ingredients Vitamin C, Vitamin E, Gingko biloba and at least one substance selected from the group consisting of a Vitamin, a mineral, an amino acid and a herb and a physiologically acceptable pharmaceutical carrier or diluent (WO 2005/117924).

In the past, the common practice to include numerous metabolically relevant ingredients in food supplements for improvement of nutrition and of the general well being has led to compositions comprising dozens of substances without verification whether these substances in combination really provide beneficial effects for the patient or not. Even worse, sometimes the erratic combination of unlimited ingredients can cause serious adverse effects in the consumer. Therefore, it becomes more and more apparent that the ingredients in such compositions should preferably not be combined at random but have to be selected carefully and based on profound knowledge.

Thus, for the prevention and/or treatment of hyperglycaemia-related diseases such as diabetes there is still a need for drugs and compositions which are easy to administer and which are pharmaceutically acceptable and have no or little adverse effects. In particular drugs are needed which do not rely or rely only to a small extent on the effects of insulin and which comprise a reasonable amount of ingredients.

Therefore, it is an object of the present invention to provide compositions effective in reducing blood glucose for the treatment of hyperglycaemia-related diseases such as diabetes.

The object is solved by the subject-matter as defined in the claims.

The following figures are part of the present invention and are included to demonstrate certain aspects of the present invention. However it has to be understood that the figures are not to be considered to be limiting the scope of the invention in any way. The invention may be better understood by reference to these figures in combination with the detailed description of specific embodiments presented herein.
Figure 1: The effects of the composition according to the present invention on blood glucose levels in four volunteers in an oral glucose tolerance test (OGTT). Blood sampling was taken from the volunteers 0, 30, 60, 90 and 120 minutes after uptake of a D-glucose solution.
Figure 2: Effects of the composition of the present invention on blood insulin levels in an oral glucose tolerance test (OGTT). Blood sampling was taken from the volunteers 0, 30, 60, 90 and 120 minutes after uptake of a D-glucose solution. IRI: immunoreactive insulin.
Figure 3: Effects of the composition according to the present invention on blood glucose levels in volunteers after consumption of 2 snack breads. Blood sampling was taken from the volunteers 0, 30, 60, 90 and 120 minutes after uptake of snack breads.
Figure 4: Effects of the composition according to the present invention on blood insulin levels after consumption of snack breads. Blood sampling was taken from the volunteers 0, 30, 60, 90 and 120 minutes after uptake of snack breads. IRI: immunoreactive insulin.
Figure 5: Comparison of the effects of L-glutamic acid vs. L-glutamine in the composition according to the present invention on blood glucose levels. IRI: immunoreactive insulin. Glutamine mix: Composition of the present invention with glutamine but without glutamic acid. Glutamic acid mix: Composition according to the present invention with glutamic acid but without glutamine. Control: OGTT without supplement.
Figure 6: Comparison of the effects of L-glutamic acid vs. L-glutamine in the composition according to the present invention on blood insulin levels. Glutamine mix: Composition of the present invention with glutamine but without glutamic acid. Glutamic acid mix: Composition according to the present invention with glutamic acid but without glutamine. IRI: immunoreactive insulin. Control: OGTT without supplement.
Figure 7: Effects of composition of the present invention on low blood glucose levels.

The term "hyperglycaemia", as used herein, refers to any elevated levels of blood glucose with regard to the normal average level of blood glucose in a human body. In particular it refers to blood glucose levels above about 100 mg/dl. The term in particular refers to blood glucose levels above about 110 mg/dl when fasting and above about 140 mg/dl two hours after having a meal.

The term "hyperglycaemia-related disease conditions", as used herein, refers to all those disease conditions which have as a symptom elevated blood glucose levels, i.e. exhibit hyperglycemia. The diesease states have in common that the elevated blood glucose levels are detrimental for the subject. The term for instance comprises the following disease conditions: several forms of diabetes, in particular Diabetes mellitus of type I and II, steroid diabetes (Morbus Cushing), gestational diabetes, secondary diabetes induced by other disease states (e.g. pancreatitis), Morbus Basedow, acromegaly, acute heart attack, hyperfunction of the adrenal gland, phaeochromocytoma, inhalation anaesthesia, shock, carbon monoxide poisoning, disorders of the central nervous system such as meningitis, craniocerebral injury, brain tumors etc. and idiopathic hyperglycaemia in new born children.

The term "diabetes", as used herein, refers to any kind of diabetes associated with high blood glucose, in particular to Diabetes mellitus of type I and II, steroid diabetes (Morbus Cushing), gestational diabetes and kinds of secondary diabetes induced by other disease states (e.g. pancreatitis).

Dosage form, as used herein, refers to an amount of medication to be taken at one time, optionally in regular intervals.

The term "supplement", "dietary supplement" or "food supplement" as used herein, refers to a composition which is consumed in addition to meals or drinks. Consumption of the supplement can occur before, simultaneously or after uptake of the meal or drinks. If consumption occurs simultaneously to the meal or drink, then the composition can be an ingredient, i.e. additive of the respective meal or drink itself, or can be consumed from a source different than the meal or drink. Ingredient, as used herein, is a substance or composition, which has been added to a composition.

The term "treatment", as used herein, comprises curing a respective disease as well as ameliorating symptoms associated with the respective disease.

According to the present invention the object is attained by a composition comprising the following substances:
Vitamin C, glutamic acid and/or glutamine, cysteine, riboflavin, succinic acid, fumaric acid, coenzyme Q10, and niacin.

Vitamin C, also called ascorbic acid is used by the body for many purposes. By far its main purpose is to function as reducing agent. For the compositions of the present invention Vitamin C (ascorbic acid) as well as its salt ascorbate can be applied.

Glutamic acid is a non-essential amino acid. It plays an important role in human metabolism and can function as neurotransmitter. Because of the latter it is often used in food supplements. For the compositions of the present invention glutamic acid as well as its salt glutamate can be applied.

Glutamine is a non-essential amino acid which plays, besides being an important component of proteins, an important role in the nitrogen metabolism. It is used as a supplement in weightlifting, bodybuilding, endurance and other sports, as well as by those who suffer from muscular cramps or pain-particularly by elderly people. For the compositions of the present invention glutamine as well as a salt of glutamine can be applied.

Cysteine is a naturally occurring amino acid which has a thiol group and is found in most proteins. When it is exposed to air, cysteine oxidizes to form cystine, which is a dimer of two cysteine molecules joined by a weak disulfide bond. Because it is a sulfur-based amino acid, cysteine itself can act as an antioxidant in the body. Cysteine is an important source of sulfur in human metabolism, and although it is classified as a non-essential amino acid, cysteine may be essential for infants, the elderly, and individuals with metabolic disease or who suffer from malabsorption syndromes. Cysteine may at some point be recognized as an essential or conditionally essential amino acid. For the compositions of the present invention cysteine as well as cystine or salts of those can be applied.

Riboflavin (Vitamin B2) is an essential compound for higher animals including humans. Riboflavin is commercially used as a vitamin preparation for use in vitamin deficiency and as a food supplement. In addition, it is also employed as a food dye, for example in mayonnaise, ice cream etc. Riboflavin can be prepared either chemically or microbiologically and can be obtained from several manufacturers. Biologically active riboflavin is flavin mononucleotide (FMN) or flavin adenine dinucleotide (FAD). These active forms and their reduced forms, FMNH₂ and FADH₂ can also be applied for use in the present invention.

Succinic acid is also termed butanedioic acid, amber acid or E 363 and is used as food supplement, for example as substitute for cooking salt in dietary meals or as flavour enhancer. In the citric cycle the salt of the succinic acid, succinate, is involved in regeneration of the acceptor oxalate. For the compositions of the present invention succinic acid as well as succinate, and also its anhydrated form succinic anhydride can be applied.

Fumaric acid, also termed 2-butenedioic acid, allomaleic acid, boletic acid or lichenic acid, is used as flavouring and thus is a common component of food additives and dietary supplements. For the compositions of the present invention fumaric acid as well as its salt fumarate can be applied.

Coenzyme Q10, also known as CoQ10 as well as ubiquinone-10 or ubiquinone 50, is the most common CoQ in human mitochondria. There it is involved in the electron transport chain. It is used as supplement because of its antioxidant function.

Niacin is also known as nicotinic acid (nicotinate) or Vitamin B3. The term also comprises the amide form, nicotinamide or niacinamide. Its biologically acitive forms nicotinamide adenine dinucleotide (NAD) and nicotinamide adenine dinucleotide phosphate (NADP) as well as their reduced forms NADH and NADPH play essential roles in the metabolism of any living being. Nicotinamide mononucleotide (NMN), desamido-NAD and deamino-NAD (also known as nicotinamide hypoxanthine dinucleotide) are metabolic intermediates containing the niacin molecule. All of these forms can be used for the compositions of the present invention. NAD, NADH, NADP, NADPH, NMN, deamido-NAD, and/or deamino-NAD containing niacin molecules can also be applied.

Peptides, i.e. polymers of amino acids are easily digested in the small intestine. Therefore, peptides consisting of cysteine, glutamic acid and/or glutamine and combinations thereof can also be applied.

In a further embodiment the present invention relates to the use of one or more of the substances selected from Vitamin C, glutamic acid and/or glutamine, cysteine, riboflavin, succinic acid, fumaric acid, coenzyme Q10, niacin, for the manufacture of a composition comprising Vitamin C, glutamic acid and/or glutamine, cysteine, riboflavin, succinic acid, fumaric acid, coenzyme Q 10 and niacin. In particular the present invention relates to the use of one or more of the substances selected from Vitamin C, glutamic acid and/or glutamine, cysteine, riboflavin, succinic acid, fumaric acid, coenzyme Q10, niacin, for the manufacture of a medicament comprising Vitamin C, glutamic acid and/or glutamine, cysteine, riboflavin, succinic acid, fumaric acid, coenzyme Q10 and niacin, in particular for manufacture of such medicament for the treatment and/or prevention of hyperglycaemia and hyperglycaemia-related disease conditions such as diabetes, in ' particular diabetes mellitus of type I and/or II, steroid diabetes (Morbus Cushing), gestational diabetes, kinds of secondary diabetes induced by other disease states (e.g. pancreatitis), Morbus Basedow, acromegaly, acute heart attack, hyperfunction of the adrenal gland, phaeochromocytoma, inhalation anaesthesia, shock, carbon monoxide poisoning, disorders of the central nervous system such as meningitis, craniocerebral injury, brain tumours, idiopathic hyperglycaemia in new born children, impaired fasting glycaemia (IFG) and/or impaired glucose tolerance (IGT).

In a further aspect the present invention relates to a food supplement comprising the composition according to the present invention. The composition can comprise further substances, such as Vitamins, amino acids or other metabolically important substances. However, in a particular preferred embodiment the food supplement comprises the composition according to the present invention but no further Vitamins, no further amino acids, no further organic acids, no further nucleotides, no polysaccharides, no oligosaccharides, no disaccharides and/or no monosaccharides, in particular no glucose. In an even more preferred embodiment the composition consists only of Vitamin C, glutamic acid and/or glutamine, cysteine, riboflavin, succinic acid, fumaric acid, coenzyme Q10, and niacin plus optionally suitable carrier substances. The use of the composition of the present invention as food supplement allows an easy way to profit from the advantageous effects of the composition of the present invention on blood glucose levels. The food composition is not restricted to human use only but can also be used for animal food, in particular pet food due to the biological importance of the substances of the composition of the present invention not only for man but for animals as well.

If the composition is to be administered as a food supplement or dietary supplementation, it is preferably an ingredient of a kind of drink, that allows the composition according to the present invention to be consumed before, simultaneously or after consumption of food or drinks, in particular after consumption of food or drinks with elevated carbohydrate content. Such drink unit can comprise a dosage of the composition according to the present invention sufficient to reduce blood glucose levels after a meal to normal levels.

Alternatively, the food supplement is consumed as tablets or capsules. Preferably, each tablet and/or capsule is so shaped and dimensioned that it allows said tablet and/or capsule to be easily swallowed. Such tablet and/or capsule can comprise a dosage sufficient to moderate blood glucose levels after a meal. But preferably said tablets and/or capsules are in such a form that one dosage includes a plurality of tablets and/or capsules. The tablets and/or capsules may be accommodated within a dosage receptacle which includes a number of those tablets and/or capsules. The food supplement or dietary supplementation may be separated into separate subunits, i.e. not all ingredients and/or substances of the composition of the present invention have to be within the same capsule or tablet. For example, it is possible to provide one unit, for example a capsule including the Vitamin C fraction, cysteine, riboflavin, succinic acid, fumaric acid and coenzyme Q10, whilst the rest of the substances of the composition of the present invention is kept in separate units, capsules, tablets or the like. However, preferably all the different subunits are consumed simultaneously or in close time intervals. The tablets or capsules can also comprise suitable carrier substances examples of which will be known to the skilled artisan.

In another embodiment the food supplement according to the present invention is of a cube type form.

The food supplement can also be in the form of a powder, in particular a cryopowder, which can be added to the meal and/or drink directly or which is dissolved for example in water prior to use.

The food supplement can also be present in form of a liquid, in particular as a syrup-type liquid, which can be added to the meal or drink, and/or which can be consumed on its own. In a particular embodiment the food supplement is dissolved in water.

If the food supplement is already an ingredient of the meal or drink, then the dosage of the composition according to the present invention is preferably calculated to moderate blood glucose levels resulting from uptake of the respective meal and/or drink.

It is possible to add further substances to the food supplement such as sweetening, flavouring, preservatives, stabilizers, colourings and pigments. Exemplary additives are fruit juice extracts, curcuma, tannin, a powder of Panax notoginseng, and Vinca rosea in suitable amounts. Oolong tea, aloe vera and spiral water algae might also be added.

A dosage of the composition of the present invention comprises niacin (Vitamin B3), Vitamin C, glutamine and/or glutamic acid, cysteine, riboflavin (Vitamin B2), succinic acid, fumaric acid, and coenzyme Q10 in physiologically relevant amounts. The food supplement can be prepared in such manner that it is compatible with a diabetic diet.

In a further aspect the present invention relates to the composition according to the present invention for use in human and veterinary medicine. In human medicine the composition of the present invention can be administered to any kind of patient in need thereof, irregardless of age and gender. In veterinary medicine for example cats, dogs, birds, cattle, horses, rodents and so forth can be treated, as these animals are often treated when suffering, for example, from diabetes. However, in principle the composition according to the present invention can be administered to any kind of animal.

In a preferred embodiment the composition of the present invention is used for the manufacture of a pharmaceutical composition for the reduction of elevated glucose levels in the blood of a subject in general, preferably for the prevention and/or treatment of hyperglycaemia and hyperglycaemia-related disease conditions such as diabetes, in particular Diabetes mellitus of type I and/or II, steroid diabetes (Morbus Cushing), gestational diabetes and kinds of secondary diabetes induced by other disease states (e.g. pancreatitis); Morbus Basedow, acromegaly, acute heart attack, hyperfunction of the adrenal gland, phaeochromocytoma, inhalation anaesthesia, shock, carbon monoxide poisoning, disorders of the central nervous system such as meningitis, craniocerebral injury, brain tumours and idiopathic hyperglycaemia in new born children and for reducing the risk for onset or progression of the aforementioned diseases by moderating the glucose metabolism within the human body.

In another preferred embodiment the composition of the present invention is used for the manufacture of a pharmaceutical composition for the treatment of impaired fasting glycaemia (IFG) and/or impaired glucose tolerance (IGT) or other pre-diabetes disease states. IFG and IGT can both precede diabetes by years. The composition of the present invention can thus be used to reduce elevated glucose levels in the blood of a subject affected by these pre-diabetic disease conditions in order to prevent onset of a true diabetic condition.

The fact, that elevated blood glucose levels, a common feature for example of the above mentioned disease states, are much more rapidly cleared from the blood when using the composition of the present invention makes the composition of the present invention ideal for the treatment of all those disease states associated with hyperglycaemia.

Pharmaceutical compositions according to the invention can be employed in the form of pharmaceutical preparations. In such an embodiment the pharmaceutical composition comprises the composition according to the present invention and additionally a pharmaceutically acceptable carrier. Such preparations are made in a manner well known in the pharmaceutical art. One preferred preparation utilizes a vehicle of physiological saline solution, but it is contemplated that other pharmaceutically acceptable carriers such as physiological concentrations of other non-toxic salts, sterile water or the like may also be used. It may also be desirable that a suitable buffer be present in the composition. Such solutions can, if desired, be lyophilized and stored in a sterile ampoule ready for reconstitution by the addition of sterile water for ready injection.

In one embodiment such pharmaceutical compositions are preferably formulated with suitable carriers. Some examples of suitable carriers, excipients, and diluents include lactose, sorbitol, mannitol, gum acacia, calcium phosphate, alginates, calcium silicate, microcrystalline cellulose, polyvinylpyrrolidone, cellulose, gelatin, syrup, methyl cellulose, methyl-and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic degradation and promote absorption such as, for example, surface active agents.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Excipients can be also those substances usually and customarily employed to formulate dosages for parenteral administration in either unit dosage or multi-dose form or for continuous or periodic infusion.

Preferably, the pharmaceutical composition according to the present invention is administered to the subject at a suitable dose. Administration of the suitable compositions may be effected by different ways, e.g. oral, intravenous, intraperitoneal, subcutaneous, intramuscular, topical or intradermal.

If the pharmaceutical composition is to be orally administered it is preferably of similar form as discussed above for the food supplement.

The pharmaceutical composition according to the present invention can be administered when the blood glucose levels are already elevated, but can also be administered in advance if the blood glucose levels are expected to rise in the near future or if any rise of the blood glucose level would be detrimental for the health and/or status of the patient. In the latter case the pharmaceutical composition according to the present invention is administered to anticipate peaks of blood glucose levels.

Preferably, the pharmaceutical composition comprising the composition according to the present invention allows for the combined administration or application with a second pharmaceutical composition or method of treatment. In case of treatment of diabetes, the second pharmaceutical composition or method of treatment can for example comprise administering inhibitors of glucose absorption e.g. alpha glucosidase inhibitors, inhibitors of glucose production in the liver e.g. biguanide, drugs improving the insulin sensitivity improving drugs e.g. thiazolidine derivatives and/or stimulators of insulin secretion e.g. sulfonyl urea and/or tenaglinid. Preferably, the pharmaceutical composition according to the present invention is also compatible with a diabetic diet or other dietary therapy approaches to reduce or prevent hyperglycaemia.

In particular the composition of the present invention can be used when a person is already treated by administering insulin. As the composition of the present invention is capable to achieve a regulation of the blood glucose level with a lower blood insulin level then normally necessary, it will help to reduce the amount of insulin which has to be administered to the patient. This reduces for example the health costs of diabetic treatments. Therefore, the pharmaceutical composition of the present invention is preferably formulated to be administered in combination with insulin.

In a preferred embodiment the composition, the food supplement or the pharmaceutical composition according to the present invention preferably comprises Vitamin C in the range from about 0.01 g to about 50 g, preferably about 0.1 g to about 10 g, preferably about 1.0 g.

In a preferred embodiment the composition, the food supplement or the pharmaceutical composition according to the present invention preferably comprises a glutamic acid or glutamine fraction in the range from about 0.01 g to about 50 g, preferably about 0.1 g to about 10 g, preferably about 1.5 g.

In a preferred embodiment the composition, the food supplement or the pharmaceutical composition according to the present invention preferably comprises a cysteine fraction in the range from about 0.01 g to about 50 g, preferably about 0.1 g to about 10 g, preferably about 500 mg.

In a preferred embodiment the composition, the food supplement or the pharmaceutical composition according to the present invention preferably comprises riboflavin in the range from about 0.001 g to about 50 g, preferably about 0.01 g to about 1 g, preferably about 40 mg.

In a preferred embodiment the composition, the food supplement or the pharmaceutical composition according to the present invention preferably comprises succinic acid in the range from about 0.001 g to about 50 g, preferably about 0.01 g to about 1 g, preferably about 100 mg.

In a preferred embodiment the composition, the food supplement or the pharmaceutical composition according to the present invention preferably comprises fumaric acid in the range from about 0.001 g to about 50 g, preferably about 0.01 g to about 1 g, preferably about 100 mg.

In a preferred embodiment the composition, the food supplement or the pharmaceutical composition according to the present invention preferably comprises a coenzyme Q10 fraction in the range from about 0.001 g to about 50 g, preferably about 0.01 g to about 1 g, preferably about 100 mg.

In a preferred embodiment the composition, the food supplement or the pharmaceutical composition according to the present invention preferably comprises niacin in the range from about 1 mg to about 30 mg, preferably about 5, 10, 15 or 20 mg.

Preferably the composition, the food supplement or the pharmaceutical composition of the present invention is formulated to be consumed by a subject in need thereof on a daily basis.

Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims which follow the examples.

The invention will now be described in more detail by way of the following non-limiting examples.

### Examples:

### Example 1: Supplement and volunteer

Ingredients of Supplement except coenzyme Q10 were purchased from Sigma Aldrich Japan (Tokyo, Japan). AQUAQ10P40 (Nissin Pharma, Tokyo Japan), which contains 40% by volume of coenzyme Q10, was used as coenzyme Q10. One gram of Vitamin C, 1.5 gram of glutamic acid, 500 mg of cysteine, 40 mg of riboflavin, 100 mg of succinic acid, 100 mg of fumaric acid, and 10 mg of niacin, and 250 mg of AQUAQ10P40 (100 mg of coenzyme Q10) were mixed. In the experiments for the comparison of the effects of glutamic acid and glutamine, glutamine was used for the supplement instead of glutamic acid in analogous manner. The supplement was taken by the volunteers with 100 ml water 20 minutes before starting with the Oral glucose tolerance test.

Four volunteers, age 26 - 48 years old (average 38 years old), weight 45 - 75 kg (average 62 kg), two female and two male participated in the experiments. All volunteers were confirmed by blood test before the experiments to have neither diabetes mellitus nor liver dysfunction.

Standard oral glucose tolerance test (OGTT) was performed using 75 gram of D-glucose dissolved in water. The experiments were conducted with volunteers which were at least for more than 7 hour on fasting conditions and consequently hungry. The D-glucose dissolved in water was consumed within 2 minutes by the volunteers. Blood was taken from the volunteers at the following time points: 0: before starting the experiment; 30, 60, 90 and 120 minutes after drinking the D-glucose solution. The blood was centrifuged immediately after sampling, and blood glucose, insulin (immunoreactive insulin: IRI), liver and kidney functions, and fat related examination factors were analyzed by BML (Shibuya, Tokyo, Japan). Statistical analysis of the results was conducted by *t*² test, and p<0.5 was defined as the level of significance. Each experiment was conducted on a different day.

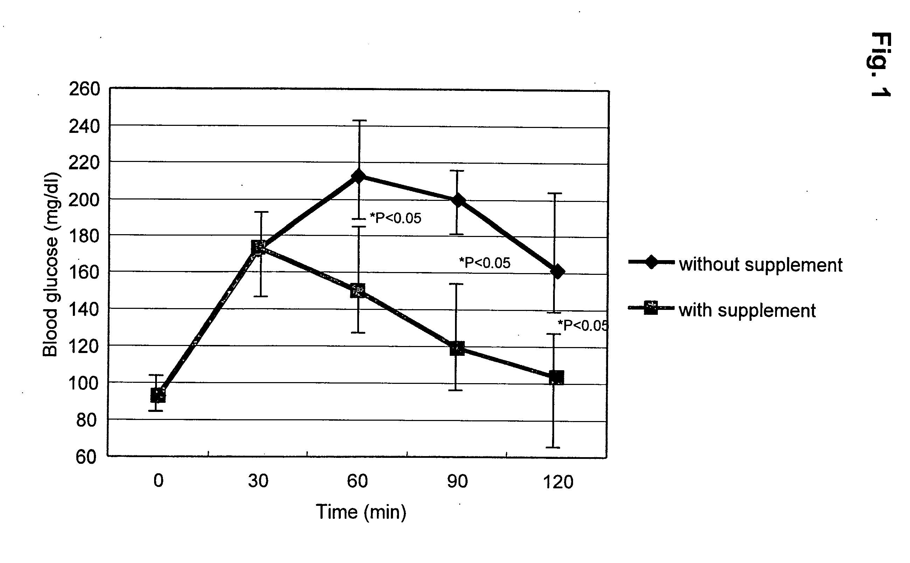

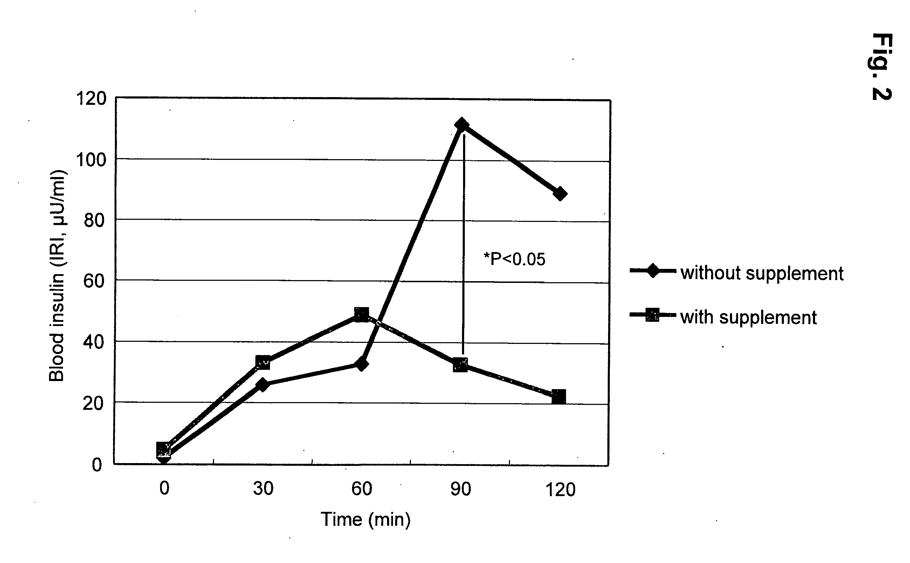

There was no significant difference between the different volunteers in liver and kidney function, and also not with regard to the fat related examination of serum. The effects of the supplement on blood glucose and insulin levels in these OGTT experiments are shown in Fig. 1 and 2, respectively. When the supplement comprising the composition according to the present invention is consumed, blood glucose levels do increase only moderately after a meal and return earlier to normal levels in comparison to subjects not having consumed the supplement. Thus, the supplement shields the human body from excessive blood glucose levels and reduces the retention time of glucose in the blood. In addition, these benefits are achieved even with lower blood insulin level, indicating that a secretion of less insulin is sufficient to clear glucose from the blood after a meal. Patients with a reduced pancreas function with regard to insulin production and/or secretion will thus benefit from the composition of the present invention.

### Example 2: Oral glucose tolerance test (OGTT) and snack bread test

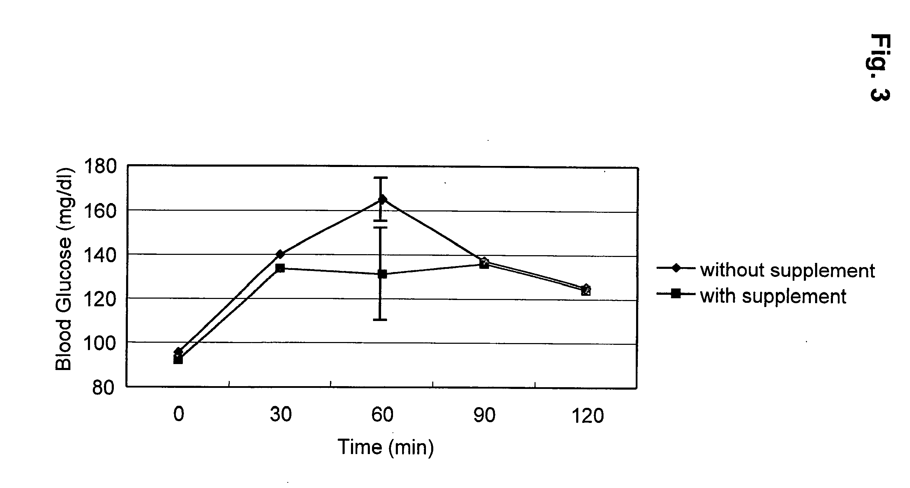

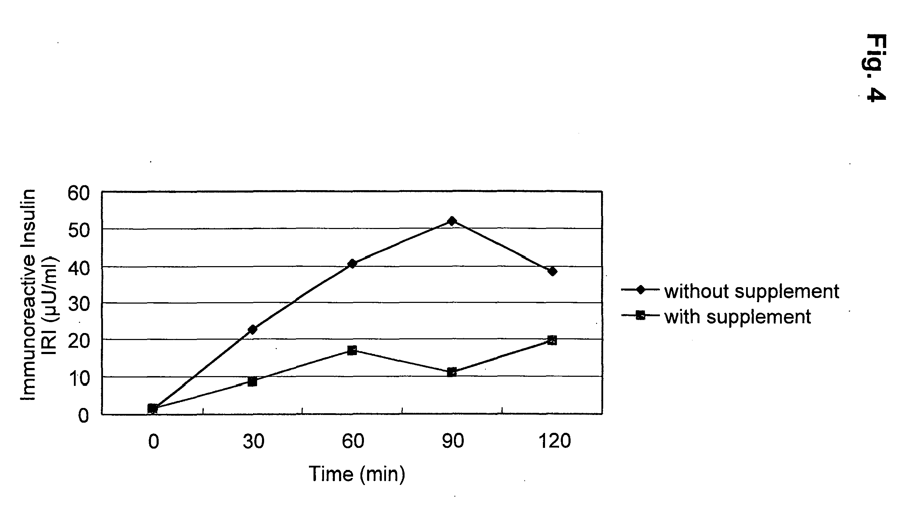

For the snack bread test, one bread with adzuki paste and one with sweet cream per person was used. Total nutrition facts of two breads are: calories: 780 Kcal, protein: 20 gram, fat: 18 gram, carbohydrate: 130 gram. Two snack breads were eaten by each volunteer within 10 minutes, and blood sampling was performed at the same time points as mentioned above. Statistical analysis of the results was conducted by *t*² test, and p<0.5 was defined as the level of significance. The effects of the supplement on blood glucose and insulin levels in the snack bread experiments are shown in Fig. 3 and 4, respectively. As above for the OGTT, the supplement comprising the composition according to the present invention provides the same advantageous effects to the consumer when consuming more complex food: Blood glucose peaks are reduced, blood glucose is cleared earlier from the blood, and less insulin is needed for glucose clearance.

### Example 3: Comparison of L-glutamic acid mix and L-glutamine mix

To examine, whether it makes any difference in effect on OGTT glucose degradation, if L-glutamic acid or L-glutamine is used in the composition of the present invention, L-glutamine was used instead of L-glutamic acid in some tests. Blood glucose and IRI was monitored in these glutamine mix experiments as well. Statistical analysis of the results was conducted by *t*² test, and p<0.5 was defined as the level of significance.

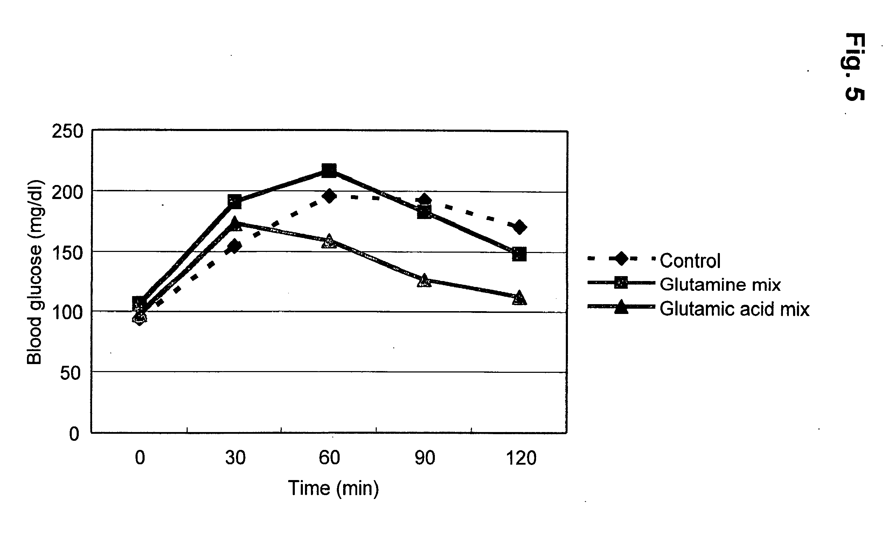

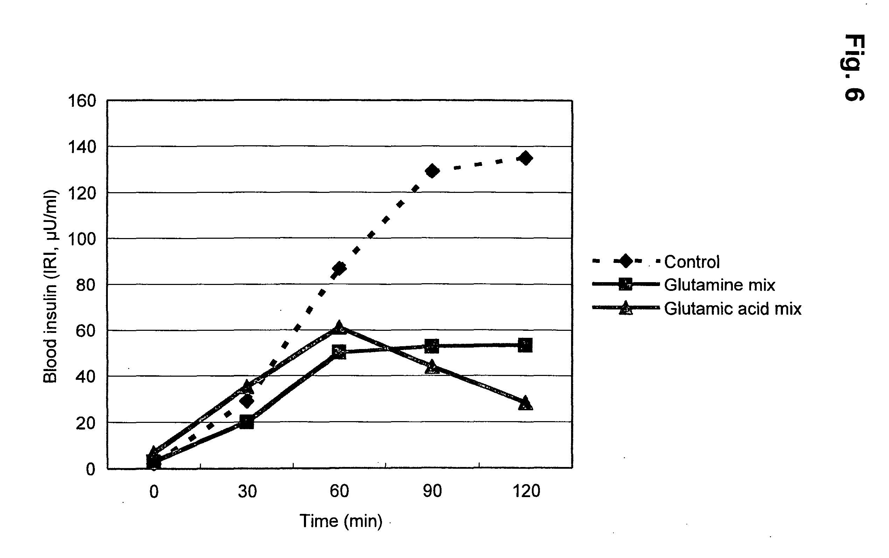

A comparison of the effects of L-glutamic acid and L-glutamine mixture on blood glucose and insulin levels is shown in Fig. 5 and 6, respectively. While the composition comprising glutamine instead of glutamic acid seems not to be as effective on absolute blood glucose levels it also provides a quicker decrease in blood glucose levels and this with less insulin in the blood.

### Example 4: Effects of supplement on blood glucose in hungry condition.

To evaluate whether or not the supplement can cause a too low blood glucose level (hypoglycaemia), the supplement was taken by volunteers in hungry condition, but without anything additional to eat or drink. Blood glucose and IRI were monitored at the same time points as for the blood sampling for the OGTT.

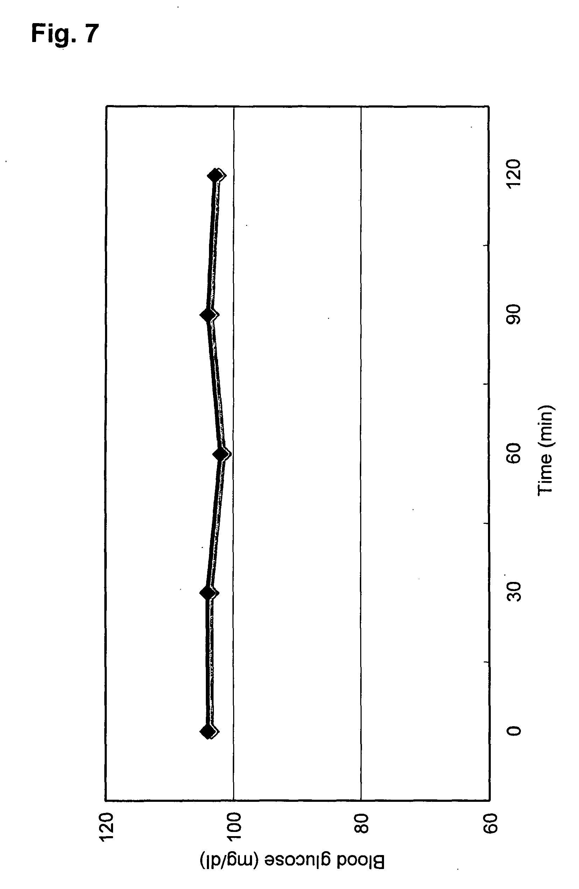

Blood glucose levels were stable throughout the experiments, and no side effects of too low blood glucose were detected. The blood insulin level was also low and stable (data not shown). This means that the supplement dose not cause a too low blood glucose level per se, and the blood glucose controlling effects of the supplement are limited to excess levels of blood glucose only. The effect of the supplement on blood glucose levels of volunteers in hungry condition is shown in Fig. 7. It is apparent, that the blood glucose levels are not affected and no hypoglycaemia is created by the composition of the present invention if the consumer is hungry, i.e. if blood glucose levels have returned to normal.

## Claims

1. Composition comprising Vitamin C, glutamic acid and/or glutamine, cysteine, riboflavin, succinic acid, fumaric acid, coenzyme Q10, and niacin.

2. Composition according to claim 1, wherein the composition comprises no glucose.

3. Composition according to anyone of the preceding claims, wherein the composition comprises a Vitamin C fraction in the range from about 0.01 g to about 50 g, preferably about 0.1 g to about 10 g, preferably about 1.0 g.

4. Composition according to anyone of the preceding claims, wherein the composition comprises an glutamic acid and/or glutamine fraction in the range from about 0.01 g to about 50 g, preferably about 0.1 g to about 10 g, preferably about 1.5 g.

5. Composition according to anyone of the preceding claims, wherein the composition comprises a cysteine fraction in the range from about 0.01 g to about 50 g, preferably about 0.1 g to about 10 g, preferably about 500 mg.

6. Composition according to anyone of the preceding claims, wherein the composition comprises a riboflavin fraction in the range from about 0.001 g to about 50 g, preferably about 0.01 g to about 1 g, preferably about 40 mg.

7. Composition according to anyone of the preceding claims, wherein the composition comprises a succinic acid fraction in the range from about 0.001 g to about 50 g, preferably about 0.01 g to about 1 g, preferably about 100 mg.

8. Composition according to anyone of the preceding claims, wherein the composition comprises a fumaric acid fraction in the range from about 0.001 g to about 50 g, preferably about 0.01 g to about 1 g, preferably about 100 mg.

9. Composition according to anyone of the preceding claims, wherein the composition comprises a coenzyme Q10 fraction in the range from about 0.001 g to about 50 g, preferably about 0.01 g to about 1 g, preferably about 100 mg.

10. Composition according to anyone of the preceding claims, wherein the composition comprises a niacin fraction in the range from about 1mg to about 30mg, preferably about 5, 10, 15 or 20 mg..

11. Use of the composition according to anyone of the preceding claims as food supplement.

12. The composition according to anyone of claims 1 to 10 for use in human and veterinary medicine.

13. Use of the composition according to anyone of claims 1 to 10 for the manufacture of a pharmaceutical composition for the treatment and/or prevention of hyperglycaemia and hyperglycaemia-related disease conditions such as diabetes, in particular diabetes mellitus of type I and/or II, steroid diabetes (Morbus Cushing), gestational diabetes, kinds of secondary diabetes induced by other disease states (e.g. pancreatitis), Morbus Basedow, acromegaly, acute heart attack, hyperfunction of the adrenal gland, phaeochromocytoma, inhalation anaesthesia, shock, carbon monoxide poisoning, disorders of the central nervous system such as meningitis, craniocerebral injury, brain tumours, idiopathic hyperglycaemia in new born children, impaired fasting glycaemia (IFG) and/or impaired glucose tolerance (IGT).

14. Composition according to anyone of claims 1 to 10, wherein the composition is present in dosage form, as powder, as liquid, in particular as drink unit or as syrup.

15. Composition according to claim 14, wherein the dosage form is in particular in form of tablets or in cube type form or wherein a dose comprises a tablet or capsule or a plurality of tablets or capsules.

16. Composition according to claim 14, wherein the tablets or capsules are present in a dosage receptacle.
